# EUROPEAN PATENT SPECIFICATION

(11) **EP 0 462 964 B1**
(45) Date of publication and mention of the grant of the patent: **22.07.1998**
(21) Application number: 89905334.2
(22) Date of filing: 13.03.1989
(51) Int. Cl.: A61K 31/19, A61K 31/23, A61K 31/20, A61K 31/195

(54) **Topical compositions containing aliphatic amines for the treatment of skin diseases**
Topische Zubereitungen enthaltend aliphatische Amine zur Behandlung von Hautkrankheiten
Compositions topiques contenant des amines aliphatiques pour le traitement de maladies de la peau

(43) Date of publication of application: 02.01.1992
(60) Divisional application: 97117896.7
(73) Proprietor: CELLEGY PHARMACEUTICALS, INC., Novato, CA 94949 (US)
(72) Inventor: Thornfeldt, Carl Richard, Ontario, Oregon 97914 (US)
(74) Representative: Sparing Röhl Henseler Patentanwälte
(86) International application number: US8901005
(87) International publication number: WO9010441

(56) References cited:
- WO-A-86/07255
- FR-A- 2 609 393
- GB-A- 2 179 249
- GB-B- 1 297 928
- GB-B- 1 410 830
- US-A- 3 987 198
- US-A- 4 406 884
- US-A- 4 478 853
- Biosis Abstracts no. 78049926
- PHARMACOL. EFF. LIPIDS 2 vol. 13, 1985, pages 77 - 87; D.V.VADEHRA ET AL.: 'Comparison of antibacterial properties of lauricidin and BHA against antibiotic resistant and sensitive strains of Staphylococcus aureus and Pseudomonas aeroginosa'

## Description

The present invention relates to a pharmaceutical composition for topical treatment of hyperactive, hyperplastic, hyperproliferative and dysplastic cells of human skin. The invention is based on the discovery that straight-chain aliphatic amines having 9 to 18 carbon atoms and pharmaceutically acceptable salts thereof effectively treat acne, rosacea, psoriasis, seborrheic dermatitis, diaper dermatitis. numular eczema, atopic eczema, contact eczema, warts, molluscum contagiosum, condylomatous carcinoma *in situ*, actinic (solar) keratoses, Bowen's disease, lentigo maligna, and melasma. These compounds may function as primary or adjunctive therapeutic agents.

U.S. Patents Nos. 4,292,326 (Nazarro-Porro, September 29, 1981) and 4,386,104 (Nazarro-Porro, May 31, 1983) and 4,713,394 (Thornfeldt, December 15, 1987) disclose the use of certain dicarboxylic acids as therapeutic agents for a variety of skin diseases. U.S. Patent No. 4,067,997 (Kabara, January 10, 1978) discloses the activity against yeast, fungus, and bacteria of a synergistic combination of a 12 carbon atom monocarboxylic acid glycerol ester and a phenolic compound, used as a food preservative.

FR-A-2609393 describes compositions for skin treatment comprising isoamylamine in amounts of 0.01-20%.

The document Biosis Abstracts no. 78049926 & Khim-Farm ZH vol. 17 no.6 1983 pages 675-679 describes bactericidal activity of higher aliphatic amine salts including cetylamine hydrochloride and dodecyclamine hydrochloride.

Acne vulgaris is a multifactorial disease occurring in teenagers and young adults, with inflammatory and noninflammatory comedos on the face and upper trunk. The disease prerequisite is sebaceous glands activated by androgens. For some yet unknown reason hypercornification in the gland duct occurs blocking normal mobility of skin and follicle microorganisms. The restricted environment stimulates release of enzymes (lipases) by Propionobacterium Acnes (an anaerobic corynebacterium), Staphylococcus Epidermidis, and Pitrosporum Ovale (a yeast). Damage to the gland structure and surrounding tissue by the lipases results in inflammatory papules, pustules, and cysts. The comedos are free of these microbes. In some, the disease is only manifest as noninflammatory lesions but all patients with inflammatory lesions have some comedos. Major treatments consist of oral and topical antibiotics and retinoids; salicylic acid, sulfur, and benzoyl peroxide topically, and oral antiandrogen birth control pills.

Psoriasis is a multifactorial disease with epidermal hyperproliferation and epidermal and dermal inflammation producing the lesions. Microbes play an etiologic role since at least 50% of the patients carry Staphylococcus Aureus in the lesions. Beta hemolytic streptococcus is known to cause guttate psoriasis. The psoriasis lesions are sharply demarcated red with thick white scale. They occur predominately on knees, elbows, scalp, genitalia, and buttocks. Current treatments consist of topical corticosteroids, tar, anthralin, methotrexate, azathioprine, etretinate, psoralens plus ultraviolet A light, and tar plus ultraviolet B light.

Eczema is a descriptive term referring to poorly demarcated pruritic, erythematous, scaley, blistered, weeping, fissured or crusted lesions due to many causes. Atopic and numular are the most common types, afflicting any age group. Usually the lesions occur on the face, neck, and flexural surfaces. In most patients, there is heavy growth of Staphylococcus Aureus from the lesions of atopic and numular eczema. A purulent rapidly progressive variant, infectious eczematoid, is due to a mixed infection of Staphylococcus Aureus and Streptococcus Pyogenes or either bacteria alone. Current therapy includes topical and systemic corticosteroids, antipruritics, and antibiotics and topical tar.

Warts and molluscum contagiosum are hyperproliferative tumors due to epidermal cell invasion by the Human Papilloma virus and a pox virus, respectively. Unlike other skin virus infections that kill the invaded cells, both these viruses produce hyperplastic, hyperproliferative keratinocytes. Both viruses most commonly infect children. The wart tumors have different morphology depending upon the viral subtype and the thickness of the skin invaded. Molluscum contagiosum are always pearly papules with a central umbilication on an erythematous base. There are currently 23 different chemical and physical destructive treatments, most of which are painful, poorly effective, or may produce systemic toxicity. Poor treatment efficacy in both infections results primarily from the marked tissue hyperplasia induced by the virus. The H.P.V. virus is a proven cancer causing agent.

Seborrheic dermatitis is a histopathologically eczematous dermatosis characterized by poorly demarcated scaley erythematous patches with yellowish greasy scales. "Dandruff" is a mild form of this condition, localized to the scalp. This disease may involve any one, several, or all of the following sites: scalp, eyebrows, glabella, paranasal and chin folds, ears and retroauricular sulci, presternal interscapular regions, pubic regions, and intergluteal folds. Pityrosporum ovale, a yeast, has been shown to play a significant role in 75% of afflicted patients. Present therapy includes corticosteroids, tar, sulfur, and antibiotics, including antiyeast agents.

Actinic keratoses are superficial inflammatory tumors arising on sun exposed and irradiated skin. These tumors are the most common premalignant skin lesions. Each tumor is erythematous to brown with variable scaling. Current therapies include excisional and cryosurgery, and 5-fluorouracil cream. The treatments hurt and often produce cosmetically unacceptable pigmentary residua.

Bowen's disease is a superficial intraepidermal tumor of keratinocytes most commonly caused by ultraviolet irradiation. Approximately 5% metastasize. These tumors frequently cover large areas of the skin. They often develop from actinic keratosis. Current treatments consist of excisional and cryosurgery and 5-fluorouracil cream.

Lentigo maligna is a tumor of premalignant melanocytes in the epidermis, usually occurring on sun exposed facial skin of elderly patients. Up to 30% progress to invasive cancer. These tumors frequently cover large surface areas. Usually treatment is surgery, although it is claimed that azelaic acid is effective in some patients.

Melasma is a hyperipigmentation state of sunexposed skin resulting from excessive hormonal stimulation of melanocytes producing pigment granule hyperplasia. This condition is usually activated by pregnancy or oral contraceptives, but may persist years after removal of this stimulus. The only current therapy is hydroquinone, which not only is poorly effective but may produce permanent depigmentation and rarely a paradoxical hyperpigmentation.

The conditions described above are the most common skin diseases and tumors, for which it has now been discovered that the claimed aliphatic amines effectively treat when applied topically. In general, this invention applies to the treatment of inflammatory and hyperproliferative skin diseases in which bacteria play a significant supporting pathophysiologic role. It also applies to treatment of tumors induced by viruses. This invention further applies to treatment of premalignant skin tumors, including Bowen's disease, lentigo maligna, actinic keratoses.

### DETAILED DESCRIPTION OF THE INVENTION AND PREFERRED EMBODIMENTS

Examples of amines salts are capratylamine hydrochloride, laurylamine hydrochloride, tridecanylamine hydrochloride, myristoleylamine hydrochloride, palmitoleylamine hydrochloride, linoleylamine hydrochloride, and linolenylamine hydrochloride. Preferred amines are dodecylamines, particularly laurylamine and laurylamine hydrochloride.

The compounds are generally applied in dermatological formulations. These include any of the various known mixtures and combinations which may be applied topically and will permit even spreading of the active ingredient over the affected area. Examples include creams, lotions, solutions, ointments, and unguents.

The concentration of the active ingredient in the formulation, i.e., aliphatic amine or its analog or salt, is not critical and may vary over a wide range. The concentration may indeed range as high as the upper limit of dissolvability in any given formulation. The concentration should be a therapeutically effective concentration, however, and in most cases, best results are achieved within a range of 1% to 35% by weight, preferably from 2.5% to 17.5% by weight.

The formulation may contain additional ingredients on an optional basis, including both those which are biologically active and those which are biologically inactive. Keratolytic agents are particularly useful in some cases as added active ingredients. Examples are salicylic acid, sulfur and retinoid derivatives. Optional concentrations will vary among keratolytic agents. Salicylic acid, for example, is preferably used at 0.5% to 5.0% while sulfur is preferably used at 2.0% to 10.0%. Appropriate concentration ranges for any particular keratolytic agent will be apparent to those skilled in the art.

Stratum corneum penetration enhancing compounds are usually included in dermatologic formulations to boost efficacy. Examples include propylene glycol, sodium lauryl sulfate, dimethylamide, N-methyl-2-pyrrolidone, and Azone® (Nelson Research, Irvine, California).

Examples of inactive ingredients are wetting agents, surfactants, emollients, and solvents.

The term "therapeutically effective amount" is used herein in terms of the amount of dermatological formulation to be applied in any particular case to denote any amount which will cause a substantial improvement in a disease condition (such as a subsidence of a lesion, for example) when applied to the affected area repeatedly over a period of time. The amount will vary with the condition being treated, the stage of advancement of the condition, and the type and concentration of formulation applied. Appropriate amounts in any given instance will be readily apparent to those skilled in the art or capable of determination by routine experimentation.

The term "pharmaceutically acceptable salts" is used herein to denote salts of the amines which are biologically compatible and otherwise suitable for administration to human subjects, and which deliver the therapeutic activity of the amine to the subject in substantially the same degree as if the amine itself were administered.

The compositions are generally applied in topical manner to the affected area, i.e., localized application to the skin region where the inflammation or hyperproliferation abnormality or tumor is manifest.

Examples 1 through 4 illustrate the preparation of topical formulations in accordance with the present invention.

### EXAMPLE 1

A therapeutic ointment was prepared by dissolving 17.5 grams of 1-monolaurin (obtained from Lauricidin, Inc., Okemos, Michigan) in 10 mℓ of commercial isopropyl alcohol heated to 50°C. Commercial propylene glycol (7 mL) was the incorporated into the solution and the resulting mixture was cooled overnight at 24°C. The mixture was then worked into 100 g of Aquaphor® (4-chloro-5-sulfamoyl-2',6'-salicyloxylidide, obtained from Beiersdorf, Inc., Norwalk Connecticut) on a pill tile.

The resulting ointment is hereinafter referred to as Formula A.

### EXAMPLE 2

A therapeutic ointment was prepared in a manner identical to that described in Example 1, except that 9 g of 1-monolaurin, 5 mℓ of isopropyl alcohol and 1 mℓ of propylene glycol were used.

The resulting ointment is hereinafter referred to as Formula B.

### EXAMPLE 3

A therapeutic formulation was prepared by dissolving 17.5 g of 1-monolaurin in 52 mℓ of isopropyl alcohol mixed with 24 mL of propylene glycol and heated to 50°C.

The resulting formulation is hereinafter referred to as Formula C.

### EXAMPLE 4

A therapeutic formulation was prepared in a manner identical to that described in Example 3, except that 24 g of 1-monolaurin, 52 mℓ of isopropyl alcohol and 24 mℓ of propylene glycol were used.

The resulting formulation is hereinafter referred to as Formula D.

Examples 5 through 10 illustrate the therapeutic effects of these formulations.

### EXAMPLE 5

Eleven patients with grade I-III acne vulgaris were treated twice daily with Formula C for 6 weeks. All these patients had failed to completely clear using all standard topical and therapeutic agents. Several had also failed using isotretinoin. With Formula C, however, seven of the patients cleared completely, two cleared more than 50% of their lesions, and two experienced mild worsening of their disease.

### EXAMPLE 6

Six patients with refractory plaque type psoriasis vulgaris were treated for four weeks twice daily with Formula A. These patients had failed to respond to all other topical and oral psoriasis treatments. As a result of the administration of Formula A, one patient completely cleared all skin lesions and the other five each experienced at least 75% clearing.

### EXAMPLE 7

Three patients with refractory facial seborrheic dermatitis were treated twice daily with Formula B for two weeks. These patients had previously failed to respond to topical corticosteroids, antifungals and antibiotics. As a result of the use of Formula B, all three gained complete resolution of the skin rash during the treatment period, and one of the three cleared in only three days.

### EXAMPLE 8

Three patients with refractory atopic dermatitis were treated with Formula A twice daily for six weeks. These patients had previously failed to respond to topical corticosteroids, tars, oral and topical antibiotics, and antihistamines. As a result of the use of Formula A, all three patients cleared completely during the treatment period.

### EXAMPLE 9

Two patients with refractory condyloma acuminata were treated with Formula D twice daily for six weeks. One completely cleared after two weeks of treatment; the other cleared after five weeks of treatment.

### EXAMPLE 10

Five patients with persistent melasma that had failed to be resolved by hydroquinone were treated for six weeks, twice daily, with Formula C. As a result, two of the five completely resolved, two others improved by 75%, and the remaining one by 50%.

## Claims

1. A pharmaceutical composition for topical treatment of hyperactive, hyperplastic, hyperproliferative and dysplastic cells of human skin, the composition containing 1% to 35% by weight with respect to the total composition of a compound, selected from the group consisting of straight-chain aliphatic amines having 9 to 18 carbon atoms and pharmaceutically acceptable salts thereof in association with a pharmaceutically acceptable vehicle that makes said composition suitable for topical application.

2. The composition of claim 1 including a stratum corneum penetration enhancer.

3. The composition of claim 1 or 2 including a keratolytic agent.

## Patentansprüche

1. Pharmazeutische Zusammensetzung zur topischen Behandlung von hyperaktiven, hyperplastischen, hyperwuchernden und dysplastischen Zellen menschlicher Haut, wobei die Zusammensetzung bezogen auf die Gesamtzusammensetzung 1 bis 35 Gew.-% einer Verbindung ausgewählt aus der Gruppe bestehend aus geradkettigen aliphatischen Aminen mit 9 bis 18 Kohlenstoffatomen und pharmazeutisch akzeptablen Salzen hiervon in Verbindung mit einem pharmazeutisch akzeptablen Träger, der die Zusammensetzung zur topischen Anwendung geeignet macht, enthält.

2. Zusammensetzung nach Anspruch 1, umfassend einen Verstärker zum Eindringen in das stratum corneum.

3. Zusammensetzung nach Anspruch 1 oder 2, umfassend ein keratolytisches Mittel.

## Revendications

1. Composition pharmaceutique pour le traitement topique des cellules hyperactives, hyperplasiques, hyperprolifératives et dysplasiques de la peau humaine, la composition contenant de 1 à 35 % en poids, par rapport à la composition totale, d'un composé choisi dans le groupe comprenant les amines aliphatiques à chaîne droite ayant de 9 à 18 atomes de carbone et leurs sels acceptables d'un point de vue pharmaceutique, en association avec un véhicule acceptable d'un point de vue pharmaceutique, qui rend ladite composition adaptée à une application topique.

2. Composition selon la revendication 1, comprenant un agent améliorant la pénétration de la couche cornée.

3. Composition selon la revendication 1 ou 2, comprenant un agent kératolytique.
